# EUROPEAN PATENT SPECIFICATION

(11) **EP 4 251 178 B1**
(45) Date of publication and mention of the grant of the patent: **09.09.2026**
(21) Application number: 21899197.4
(22) Date of filing: 30.11.2021
(51) Int. Cl.: A61K 31/5585, A61K 40/11, A61K 40/31, A61P 35/00

(54) **CAR T-CELL ADJUVANT THERAPIES**
CAR-T-ZELL-ADJUVANSTHERAPIEN
THÉRAPIES ADJUVANTES PAR CELLULES CAR-T

(30) Priority: 30.11.2020 US 202063119085 P
(43) Date of publication of application: 04.10.2023
(73) Proprietor: Cytoagents, Inc., Pittsburgh, PA 15212 (US)
(72) Inventor: CRAIGO, Jodi, Cranberry Township, Pennsylvania 16066 (US)
(74) Representative: HGF
(86) International application number: PCT/US2021/061066
(87) International publication number: WO 2022/115730

(56) References cited:
- WO-A1-2017/046747
- WO-A1-2021/222781
- US-A1- 2014 275 237

## Description

### CROSS-REFERENCE TO RELATED APPLICATIONS

### BACKGROUND

Chimeric antigen receptor T-cells (CAR T-cells) are genetically engineered T-cells that produce a receptor having the ability to bind to a specific protein. CAR T-cell therapies are attractive for use in immunotherapy as they can be designed and engineered to target and recognize cancer cells specifically.

T-cells are harvested and are genetically engineered prior to infusing back into the patient to attack their cancer cells. The T-cells can be obtained from the patient's blood ("autologous") or obtained from another donor ("allogenic"). The chimeric antigen receptor is specific for an antigen that is present on the surface of cancer cells in a tumor, but not expressed on the surface of healthy cells.

One common but serious side effect of CAR T-cell therapy is cytokine release syndrome ("CRS"). The CAR T-cells cause the release of large amounts of cytokines into the blood. This can cause high fever, low blood pressure, fatigue, headache, myalgia, nausea, capillary leakage, tachycardia, and potentially liver failure and kidney damage. CRS occurs in 50-100% of patients.

For example, with the Kymriah product from Novartis (Kymriah is a registered trademark of Novartis AG; Basel, Switzerland), CRS occurred in 79% of acute lymphocytic leukemia and 74% patients with relapsed / refractory diffuse large B cell lymphoma receiving KYMRIAH, including greater than or equal to grade 3 in 49% of patients with relapsed / refractory acute lymphocytic leukemia and in 23% of patients with relapsed / refractory diffuse large B cell lymphoma. The median time to onset was 3 days, and the median time to resolution was 8 days (a range of 1-36 days). Similarly, with the Yescarta product from Kite / Gilead (Yescarta is a registered trademark of Kite Pharma, Inc.; Santa Monica, CA, USA), CRS occurred in 94% of patients, with 13% greater or equal to Grade 3. Among patients who died after receiving Yescarta, 4 had ongoing CRS at death. The median time to onset was 2 days (range: 1-12 days) and median duration was 7 days (range: 2-58 days).

From one perspective, the development of CRS is an indicator or diagnostic marker that the CAR T-cells are functioning to attack cancer cells. CRS develops in a delayed time after initiation of CAR T-cell administration. This lag time is very predictable and consistent.

CAR T-cell therapies are powerful and effective "last resort" therapies, but there exists a need for new and improved treatments to reduce or eliminate the undesired and potentially fatal CRS side effects.

### SUMMARY

Disclosed herein are compositions to administer CAR T-cell therapies while reducing or eliminating CRS side effects.

Methods of treating cancer generally comprise administering CAR T-cells and as an adjuvant therapy / combination therapy pharmaceutical composition which comprises an effective amount of prostacyclin/prostaglandin analog BPS-314d (GP1681, esuberaprost sodium salt).

Kits for the treatment of cancer in a subject can generally comprise a first container containing a pharmaceutical composition comprising at least an effective amount of beraprost or a pharmaceutically acceptable salt thereof; a second container containing CAR T-cells; and instructions for the administration of the pharmaceutical composition and CAR T-cells to the subject.

### DEFINITIONS

As used herein, the term "about" when immediately preceding a numerical value means a range of plus or minus 10% of that value, e.g., "about 50" means 45 to 55, "about 25,000" means 22,500 to 27,500, etc., unless the context of the disclosure indicates otherwise, or is inconsistent with such an interpretation.

As used herein the term "analog" refers to a compound, the presence of which results in a biological activity of a receptor that is the same as the biological activity resulting from the presence of a naturally occurring ligand for the receptor.

The terms "administer," "administering" or "administration" as used herein refer to directly administering a compound or a composition to a subject.

As used herein, the term "effective amount" refers to an amount that results in measurable inhibition of at least one symptom or parameter of a specific disorder or pathological process. As used herein the term "therapeutically effective amount" of compositions of the application is an amount, which confers a therapeutic effect on the treated subject, at a reasonable benefit/risk ratio applicable to any medical treatment. The therapeutic effect may be objective (that is, measurable by some test or marker) or subjective (that is, subject gives an indication of or feels an effect or physician observes a change).

As used herein the term "immediate release" refers to pharmaceutical compositions that release the active ingredient within a short period of time.

As used herein the term "modified release" refers to pharmaceutical compositions that does not otherwise release the active ingredient immediately, for example it may release the active ingredient at a sustained or controlled rate over an extended period of time, or may release the active ingredient after a lag time after administration, or may be used optionally in combination with an immediate release composition. Modified release includes extended release, sustained release, and delayed release. The term "extended release" or "sustained release" as used herein is a dosage form that makes a drug available over an extended period of time after administration. The term "delayed release" as used herein is a dosage form that releases a drug at a time other than immediately upon administration.

The phrase "pharmaceutically acceptable salt(s)", as used herein, includes those salts of compounds of the application that are safe and effective for use in mammals and that possess the desired biological activity. Pharmaceutically acceptable salts include salts of acidic or basic groups present in compounds of the application or in compounds identified pursuant to the methods of the application. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., l, l'-methylene-bis-(2-hydroxy-3-naphthoate)) salts. Certain compounds of the application can form pharmaceutically acceptable salts with various amino acids. Suitable base salts include, but are not limited to, aluminum, calcium, lithium, magnesium, potassium, sodium, zinc, iron and diethanolamine salts. Pharmaceutically acceptable base addition salts are also formed with amines, such as organic amines. Examples of suitable amines are N,N'-dibenzylethylenediamine, chloroprocaine, choline, diethanolamine, dicyclohexylamine, ethylenediamine, N-methylglucamine, and procaine.

The term "preventing" may be taken to mean to prevent a specific disorder, disease, or condition and/or prevent the reoccurrence of a specific disorder, disease, or condition.

The term "substantially pure isomer" refers to a formulation or composition wherein among various isomers of a compound a single isomer is present at 70%, or greater or at 80% or greater, or at 90% or greater, or at 95% or greater, or at 98% or greater, or at 99% or greater, or said compound or composition comprise only a single isomer of the compound.

As used herein the terms "treat", "treated", or "treating" refer to both therapeutic treatment and prophylactic or preventative measures, wherein the object is to protect against (partially or wholly) or slow down (for example, lessen or postpone the onset of) an undesired physiological condition, disorder or disease, or to obtain beneficial or desired clinical results such as partial or total restoration or inhibition in decline of a parameter, value, function or result that had or would become abnormal. For the purposes of this application, beneficial or desired clinical results include, but are not limited to, alleviation of symptoms; diminishment of the extent or vigor or rate of development of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether or not it translates to immediate lessening of actual clinical symptoms, or enhancement or improvement of the condition, disorder or disease. Treatment seeks to elicit a clinically significant response without excessive levels of side effects.

The term "unit dosage form" refers to physically discrete units suitable as a unitary dosage for human subjects and other animals, each unit containing a predetermined quantity of active material calculated to produce the desired therapeutic effect, in association with a suitable pharmaceutical excipient.

The "weight percent" disclosed herein may be weight-to-weight percent or weight-to-volume percent, depending upon the composition.

The term "disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "combination therapy" means the administration of two or more therapeutic agents to treat a medical condition or disorder. Such administration encompasses co-administration of these therapeutic agents in a substantially simultaneous manner, such as in a single capsule, or dosage presentation, having a fixed ratio of active ingredients or in multiple, separate capsules for each active ingredient. In addition, such administration also encompasses use of each type of therapeutic agent in a sequential manner in the same patient, with delivery of the individual therapeutics separated by 1-24 hours, 1-7 days, or 1 or more weeks. In either case, the treatment regimen will provide beneficial effects of the drug combination in treating the conditions or disorders described herein.

The present disclosure is not to be limited in terms of the particular embodiments described in this application, which are intended as illustrations of various aspects. Many modifications and variations can be made without departing from its spirit and scope, as will be apparent to those skilled in the art. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds, compositions, or biological systems, which can, of course, vary. It is also to be understood that the terminology used herein is for the purpose of describing particular embodiments only, and is not intended to be limiting.

As used in this document, the singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention. As used in this document, the term "comprising" means "including, but not limited to."

While various compositions, methods, and devices are described in terms of "comprising" various components or steps (interpreted as meaning "including, but not limited to"), the compositions, methods, and devices can also "consist essentially of" or "consist of" the various components and steps, and such terminology should be interpreted as defining essentially closed-member groups.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (for example, bodies of the appended claims) are generally intended as "open" terms (for example, the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (for example, "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (for example, the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (for example, " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (for example, " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

### DETAILED DESCRIPTION

This disclosure is not limited to the particular systems, devices and methods described, as these may vary. The terminology used in the description is for the purpose of describing the particular versions or embodiments only, and is not intended to limit the scope.

Various methods and kits are described herein for performing CAR T-cell therapies with reduced or eliminated CRS effects in a subject. The methods can include administration of CAR T-cells and at least one pharmaceutical composition to the subject. The T-cells and T-cell therapy can be autologous or allogenic.

### Types of cancers

The cancer can generally be any cancer suitable for treatment with CAR T-cell therapy. For example, the cancer can be B-cell lymphoma, aggressive, relapsed or refractory diffuse large B cell lymphoma, primary mediastinal B-cell lymphoma, high grade B-cell lymphoma, transformed follicular lymphoma, relapsed or refractory mantle cell lymphoma, acute lymphoblastic leukemia, Hodgkin's lymphoma, non-Hodgkin's lymphoma, acute myeloid leukemia, or multiple myeloma. In some examples, the cancer is B-cell lymphoma. Other cancers suitable for treatment with CAR T-cell therapy include brain cancer, breast cancer, glioblastoma, lung cancer, non-small-cell lung cancer, multiple myeloma, ovarian cancer, neuroblastoma, colorectal, biliary, pancreatic, mesothelioma, hepatoblastoma, embryonal sarcoma, prostate, sarcoma, and liver metastases.

### Medical uses for treating cancer

The compounds and pharmaceutical compositions described herein may be administered at therapeutically effective dosage levels to treat the recited conditions, disorders, and diseases.

The compounds and pharmaceutical compositions described herein may be administered at prophylactically effective dosage levels to mitigate or prevent the recited conditions, disorders, and diseases.

Use of the described methods and pharmaceutical compositions can result in a reduction or elimination of CRS. Reduction can be an improvement or resolution of undesirable physiological symptoms the patient subject is experiencing, a quantifiable reduction in one or more cytokine concentration, or both. The reduction can generally be reduced by any amount. For example, the reduction can be at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, at least about 99%, and in an ideal situation, about 100% reduction (complete elimination of disease, symptom, or other undesired property). Reduction can be relative to CRS that would be observed with administration of the CAR T-cells but without administration of the pharmaceutical composition.

CAR T-cells are typically delivered by infusion in one single administration, although multiple administrations are also possible. While CRS does not occur in all patients, about 50-100% of patients receiving CAR T-cell therapy do develop CRS. CRS typically has an onset within the first week and can typically occur over the first two weeks post administration of CAR T-cells. The pharmaceutical composition can be administered starting concurrently with the CAR T-cells (that is, no delay period), or starting after a delay period. In some examples, the pharmaceutical composition can additionally be administered one or more times prior to administration of the CAR T-cells. The delay period can be a predetermined period of time after administration of the CAR T-cells (such as about 1 day, about 2 days, about 3 days, about 4 days, about 5 days, about 6 days, about 7 days, about 8 days, about 9 days, about 10 days, about 11 days, about 12 days, about 13 days, about 14 days, or more, or ranges between any two of these values). Example ranges of the delay period include about 3 days to about 7 days, about 2 days to about 5 days, about 3 days to about 5 days, and so on. Alternatively, the delay period can last until onset of CRS is detected.

Onset of CRS can be detected by generally any method, such as detecting fever, headache, anorexia, nausea, fatigue, myalgia, hypoxia, low blood pressure, hypotension, impaired coagulation, capillary leakage, tachycardia, organ system failure and so on. For example, a simple method to detect onset of CRS is detecting fever. Alternatively, onset of CRS can be detected by monitoring increased levels of one or more cytokines such as IL-1α, IL-β, IL-2, IL-4, IL-6, IL-7, IL-8, IL-10, IL-12, IFN-y, TNF-α, IP-10, MCP-1, MIP-1, RANTES, and GM-CSF. In some examples, onset of CRS can be detected by monitoring increased levels of one or more cytokines such as IL-6, IFN-y, TNF-α, and IL-10.

For example, after the delay period or upon detecting onset of CRS, the pharmaceutical composition can be administered for about 1 day, 2 days, 3 days, 4 days, 5 days, 6 days, 7 days, 8 days, 9 days, 10 days, 11 days, 12 days, 13 days, 14 days, or longer, or ranges between any two of these values. CRS typically is resolved in about one week but has been documented to persist for 30 days or beyond. In some examples, the pharmaceutical composition can be administer for more than 14 days, such as 15 days, 16 days, 17 days, 18 days, 19 days, 20 days, 21 days, 22 days, 23 days, 24 days, 25 days, 26 days, 27 days, 28 days, 29 days, 30 days, or longer, or ranges between any two of these values. Example ranges include about 1 day to about 7 days, about 1 day to about 14 days, about 1 day to about 21 days, about 1 day to about 30 days, about 7 days to about 14 days, about 7 days to about 21 days, about 7 days to about 30 days, about 14 days to about 21 days, and about 21 days to about 30 days.

The treatments can generally be performed at any effective schedule. For example, the pharmaceutical compositions disclosed herein may be administered once, as needed, once daily, twice daily, three times a day, four times a day, once a week, twice a week, three times a week, four times a week, five times a week, six times a week, seven times a week, every other week, every other day, or the like for one or more dosing cycles. It will be understood that the specific dose level and frequency of dosage for any particular subject can be varied and will depend upon a variety of factors including the species, age, body weight, general health, gender and diet of the subject, the mode and time of administration, rate of excretion, drug combination, and severity of the particular condition.

Administration may be performed by generally any method. Example delivery methods of administering include topical delivery, subcutaneous delivery, intravenous injection (IV) delivery, intramuscular injection (IM) delivery, intrathecal injection (IT) delivery, intraperitoneal injection (IP) delivery, transdermal delivery, subcutaneous delivery, oral delivery, transmucosal oral delivery, pulmonary delivery, inhalation delivery, intranasal delivery, buccal delivery, rectal delivery, vaginal delivery, and combinations thereof. In some examples, the administering comprises oral delivery, subcutaneous, inhalation, IV, or IM.

### Pharmaceutical compositions

The pharmaceutical composition comprise the prostacyclin / prostaglandin analog BPS-314d, also known as GP1681, or esuberaprost sodium salt (C₂₄H₂₉NaO₅; 2,3,3a,8b-tetrahydro-2-hydroxyl-1-(3-hydroxyl-4-methyl-1-octen-6-ynyl)-1H-cyclopenta[b]benzofuran-5-butanoic acid, sodium salt). Beraprost isomers are further described in U.S. Patent Publication No. US 2014-0275237 A1.

Beraprost and methods for its preparation are shown in U.S. Patent No. 7,345,181 and PCT Publication No. WO 2004/026224, entitled "Process for preparing prostaglandin derivatives and starting materials for the same". Beraprost is commercially available from Yonsung Fine Chemicals (Gyeonggi-do, Republic of Korea). The beraprost can be present in the pharmaceutical composition at generally any effective amount or effective concentration. Different pharmaceutical forms may have different amounts or concentrations of beraprost.

The daily dose (mass) of prostacyclin/prostaglandin analog or beraprost or pharmaceutically acceptable salt thereof can generally be any effective amount or dosage. For example, the therapeutically effective amount (in micrograms) may include about 0.1 µg to about 100 µg, about 10 µg to about 90 µg, or about 15 µg to about 90 µg. The mass values are the combined salt weight, that is the anion and cation together. Specific examples of therapeutically effective amounts include about 0.1 µg, about 1 µg, about 10 µg, about 20 µg, about 30 µg, about 40 µg, about 50 µg, about 60 µg, about 70 µg, about 80 µg, about 90 µg, about 100 µg, and ranges between any two of these values. When administered in two or more daily doses, the amount in each dose can be added together to yield a total daily dose. For example, GP1681 may be administered at a dose of about 15-90 µg/day divided into 3 doses, and each individual dose of about 5-30 µg.

In some embodiments, the effective amount of beraprost or an isomer thereof or a pharmaceutically acceptable salt thereof are present in a unit dose (mass) of the pharmaceutical composition is at least about 1 microgram, about 1 microgram to about 100 micrograms, about 1 microgram to about 80 micrograms, about 1 microgram to about 60 micrograms, about 1 microgram to about 50 micrograms, about 1 microgram to about 40 micrograms, about 51 microgram to about 30 micrograms, about 1 microgram to about 20 micrograms, about 1 mg to about 10 micrograms, or about 1 microgram to about 5 micrograms, or any value between these ranges. Specific examples include about 1 microgram, about 5 micrograms, about 10 micrograms, about 25 micrograms, about 50 micrograms, about 75 micrograms, about 100 micrograms, or ranges between any two of these values.

In some embodiments, the amount of beraprost or an isomer thereof or a pharmaceutically acceptable salt thereof can be calculated based on the presence of a single desired isomer. For example, if the single isomer BPS-314d (GP1681; also esuberaprost sodium salt) is desired at an amount of about 15 micrograms to about 90 micrograms, this is equivalent to an amount of about 60 micrograms to about 360 micrograms of a racemic mixture of four isomers (where the amount of a single isomer is one-quarter of the mass).

In some embodiments, the pharmaceutical composition comprising beraprost or an isomer thereof or a pharmaceutically acceptable salt thereof achieves a Cmax of about 0.01 nanomolar to about 10 nanomolar, about 0.01 nanomolar to about 5 nanomolar, about 0.01 nanomolar to about 3 nanomolar, about 0.01 nanomolar to about 2 nanomolar, about 0.01 nanomolar to about 1 nanomolar, about 0.01 nanomolar to about 0.5 nanomolar, or any values between these ranges. Specific examples include about 0.01 nanomolar, about 0.05 nanomolar, about 0.075 nanomolar, about 0.1 nanomolar, about 0.5 nanomolar, about 1 nanomolar, about 2 nanomolar, about 5 nanomolar, or about 10 nanomolar.

In some embodiments, the pharmaceutical composition comprising beraprost or an isomer thereof or a pharmaceutically acceptable salt thereof achieves a Tmax at about 0.1 hour to about 5 hours, about 0.1 hour to about 4 hours, about 0.1 hour to about 3 hours, about 0.1 hour to about 2 hours, about 0.1 hour to about 1 hours, or any specific value between these ranges. Specific examples include about 0.1 hour, about 0.5 hour, about 1 hour, about 1.5 hours, about 1.7 hours, about 2 hours, or about 5 hours.

In some embodiments, the pharmaceutical composition comprising beraprost or an isomer thereof or a pharmaceutically acceptable salt thereof achieves an AUC of about 0.01 ng.hr/mL to about 30 ng.hr/mL over a 48 hour period, about 0.01 ng.hr/mL to about 20 ng.hr/mL over a 48 hour period, about 0.01 ng.hr/mL to about 10 ng.hr/mL over a 48 hour period, about 0.01 ng.hr/mL to about 5 ng.hr/mL over a 48 hour period, about 0.01 ng.hr/mL to about 3 ng.hr/mL over a 48 hour period, about 0.01 ng.hr/mL to about 2 ng.hr/mL over a 48 hour period, or about 0.01 ng.hr/mL to about 1 ng.hr/mL over a 48 hour period. Specific examples include about 0.01 ng.hr/mL, about 0.05 ng.hr/mL, about 0.1 ng.hr/mL, about 0.5 ng.hr/mL, about 1 ng.hr/mL, about 2 ng.hr/mL, about 5 ng.hr/mL, about 10 ng.hr/mL, or about 30 ng.hr/mL.

In some examples, the pharmaceutical composition can further comprise at least one anti-inflammatory component such as at least one corticosteroid or at least one therapeutic monoclonal antibody.

In some examples, the pharmaceutical composition further comprises one or more pharmaceutically acceptable excipients. Examples of pharmaceutically acceptable excipients that may be present in the composition include but not limited to fillers/vehicles, solvents/co-solvents, preservatives, antioxidants, suspending agents, surfactants, antifoaming agents, buffering agents, chelating agents, sweeteners, flavoring agents, binders, extenders, disintegrants, diluents, lubricants, fillers, wetting agents, glidants, and combinations thereof.

In some examples, the pharmaceutic composition can further comprise one or more exemplary fillers. Examples of exemplary fillers include cellulose and cellulose derivatives such as microcrystalline cellulose; starches such as dry starch, hydrolyzed starch, and starch derivatives such as com starch; cyclodextrin; sugars such as powdered sugar and sugar alcohols such as lactose, mannitol, sucrose and sorbitol; inorganic fillers such as aluminum hydroxide gel, precipitated calcium carbonate, carbonate, magnesium aluminometasilicate, dibasic calcium phosphate; and sodium chloride, silicon dioxide, titanium dioxide, titanium oxide, dicalcium phosphate dihydrate, calcium sulfate, alumina, kaolin, talc, or combinations thereof. Fillers may be present in the composition from about 20 wt% to about 65 wt%, about 20 wt% to about 50 wt%, about 20 wt% to about 40 wt%, about 45 wt% to about 65 wt%, about 50 wt% to about 65 wt%, or about 55 wt% to about 65 wt% of the total weight of the composition, or any value between these ranges.

In some examples, the pharmaceutical composition further comprises one or more disintegrants. Examples of disintegrants include starches, alginic acid, crosslinked polymers such as crosslinked polyvinylpyrrolidone, croscarmellose sodium, potassium starch glycolate, sodium starch glycolate, clays, celluloses, starches, gums, or combinations thereof. Disintegrants may be present in the composition from about 1 wt% to about 10 wt%, about 1 wt% to about 9 wt%, about 1 wt% to about 8 wt%, about 1 wt% to about 7 wt%, about 1 wt% to about 6 wt%, or about 1 wt% to about 5 wt% of the total weight of the composition, or any value between these ranges.

In some examples, the pharmaceutical composition further comprises one or more binders, including but not limited to celluloses such as hydroxypropylcellulose, methyl cellulose, and hydroxypropylmethylcellulose; starches such as corn starch, pregelatinized starch, and hydroxpropyl starch; waxes and natural and synthetic gums such as acacia, tragacanth, sodium alginate; synthetic polymers such as polymethacrylates and polyvinylpyrrolidone; and povidone, dextrin, pullulane, agar, gelatin, tragacanth, macrogol, or combinations thereof. Binders may be present in the composition from about 0.5 wt% to about 5 wt%, about 0.5 wt% to about 4 wt%, about 0.5 wt% to about 3 wt%, about 0.5 wt% to about 2 wt%, or about 0.5 wt% to about 1 wt% of the total weight of the composition, or any value between these ranges.

In some examples, the pharmaceutical composition further comprises one or more wetting agents, including but not limited to oleic acid, glyceryl monostearate, sorbitan mono-oleate, sorbitan monolaurate, triethanolamine oleate, polyoxyethylene sorbitan mono-oleate, polyoxyethylene sorbitan monolaurate, sodium oleate, sodium lauryl sulfate, poloxamers, poloxamer 188, polyoxyethylene ethers, polyoxyethylene sorbitan fatty acid esters, polyoxyethylene fatty acid esters, polyethylene glycol fatty acid esters, polyoxyethylene hardened castor oil, polyoxyethylene alkyl ethers, polysorbates, cetyl alcohol, glycerol fatty acid esters (e.g., triacetin, glycerol monostearate, etc.), polyoxymethylene stearate, sodium lauryl sulfate, sorbitan fatty acid esters, sucrose fatty acid esters, benzalkonium chloride, polyethoxylated castor oil, and combinations thereof. Wetting agents may be present in the composition from about 0.1 wt% to about 1 wt%, about 0.1 wt% to about 2 wt%, about 0.1 wt% to about 3 wt%, about 0.1wt% to about 4 wt%, or about 0.1 wt% to about 5 wt% of the total weight of the composition, or any value between these ranges.

In some examples, the pharmaceutical composition further comprises one or more lubricants, including but not limited to stearic acid, magnesium stearate, calcium hydroxide, talc, com starch, sodium stearyl fumarate, alkali-metal and alkaline earth metal salts, waxes, boric acid, sodium benzoate, sodium acetate, sodium chloride, leucine, polyethylene glycol (PEG), a methoxypolyethylene glycol, propylene glycol, sodium oleate, glyceryl behenate, glyceryl palmitostearate, glyceryl benzoate, magnesium lauryl sulfate, sodium lauryl sulfate, and combinations thereof. Lubricants may be present in the composition from about 0.1 wt% to about 5 wt%, about 0.1 wt% to about 4 wt%, about 0.1 wt% to about 3 wt%, about 0.1 wt% to about 2 wt%, or about 0.1 wt% to about 1 wt% of the total weight of the composition, or any value between these ranges.

In some examples, the pharmaceutical composition further comprises one or more glidants, including but not limited to colloidal silicon dioxide, talc, sodium lauryl sulfate, native starch, and combinations thereof. Glidants may be present in the composition from about 0.05 wt% to about 1 wt%, about 0.05 wt% to about 0.9 wt%, about 0.05 wt% to about 0.8 wt%, about 0.05 wt% to about 0.5 wt%, or about 0.05 wt% to about 0.1 wt% of the total weight of the composition, or any value between these ranges.

In some examples, the pharmaceutical composition is a tablet and further comprises a top coat, such as hydroxypropyl-methylcellulose coating or polyvinyl alcohol coating, and are available under the trade name Opadry, such as Opadry White, Opadry II (Opadry is a registered trademark of BPSI Holdings LLC, Wilmington, DE, USA). Top coats may be present in the composition from about 1 wt% to about 10 wt%, about 1 wt% to about 9 wt%, about 1 wt% to about 8 wt%, about 1 wt% to about 7 wt%, about 1 wt% to about 6 wt%, or about 1 wt% to about 5 wt% of the total weight of the composition, or any value between these ranges.

In some examples, the pharmaceutical composition can further comprise one or more preservative agents. Examples of preservative agents include sodium benzoate, paraoxybenzoic acid esters, methyl, ethyl, butyl, and propyl parabens, chlorobutanol, benzyl alcohol, phenylethylalcohol, dehydroacetic acid, sorbic acid, benzalkonium chloride (BKC), benzethonium chloride, phenol, phenylmercuric nitrate, thimerosal, or combinations thereof. Preservative agents can be included in the liquid dosage form. The preservative agents can be in an amount sufficient to extend the shelf-life or storage stability, or both, of the liquid dosage form. Preservatives may be present in the composition from about 0.05 wt% to about 1 wt%, about 0.05 wt% to about 0.9 wt%, about 0.05 wt% to about 0.8 wt%, about 0.05 wt% to about 0.5 wt%, or about 0.05 wt% to about 0.1 wt% of the total weight of the composition, or any value between these ranges.

In some examples, the pharmaceutical composition can further comprise one or more flavoring agents. Examples of flavoring agents include synthetic flavor oils and flavoring aromatics and/or natural oils, extracts from plants leaves, flowers, fruits, and so forth and the like or any combinations thereof. Additional examples include cinnamon oil, oil of wintergreen, peppermint oils, clove oil, bay oil, anise oil, eucalyptus, thyme oil, cedar leaf oil, oil of nutmeg, oil of sage, oil of bitter almonds, and cassia oil and the like or any combinations thereof. Also useful as flavors are vanilla, citrus oil, including lemon, orange, grape, lime and grapefruit, and fruit essences, including apple, banana, pear, peach, strawberry, raspberry, cherry, plum, pineapple, apricot, strawberry flavor, tutti-fruity flavor, mint flavor, or any combinations thereof. Flavoring agents may be present in the composition from about 0.1 wt% to about 5 wt%, about 0.1 wt% to about 4 wt%, about 0.1 wt% to about 3 wt%, about 0.1 wt% to about 2 wt%, or about 0.1 wt% to about 1 wt% of the total weight of the composition, or any value between these ranges.

In some examples, the pharmaceutical composition can further comprise one or more antioxidants. Examples of antioxidants include flavonoids, anthocyanidins, anthocyanins, proanthocyanidins, or combinations thereof. Antioxidants may be present in the composition from about 0.05 wt% to about 1 wt%, about 0.05 wt% to about 0.9 wt%, about 0.05 wt% to about 0.8 wt%, about 0.05 wt% to about 0.5 wt%, or about 0.05 wt% to about 0.1 wt% of the total weight of the composition, or any value between these ranges.

### Physical form of the pharmaceutical composition

The pharmaceutical compositions can generally be in any physical form suitable for use in treating a subject. These forms can be referred to as a unit dosage form, such as an individual pill or tablet. In some examples, the pharmaceutical compositions can be formulated as tablets, capsules, granules, powders, liquids, suspensions, gels, syrups, slurries, suppositories, patches, nasal sprays, aerosols, injectables, implantable sustained-release formulations, or mucoadherent films. In some examples, the pharmaceutical composition may be formed as a tablet, a bi-layer tablet, a capsule, a multiparticulate, a drug coated sphere, a matrix tablet, or a multicore tablet. A physical form can be selected according to the desired method of treatment. In some examples, the physical form can be a liquid, for example for oral or IV, IP, IM, or IT administration.

Pharmaceutical compositions can be manufactured by various conventional methods such as conventional mixing, dissolving, granulating, dragee-making, levigating, emulsifying, encapsulating, entrapping, or lyophilizing processes. Pharmaceutical compositions can be formulated in a conventional manner using one or more physiologically acceptable carriers, diluents, excipients, or auxiliaries that facilitate processing of the active agent into preparations that can be used pharmaceutically. Proper formulation can be selected upon the route of administration chosen.

For topical administration the pharmaceutical compositions described herein may be formulated as solutions, gels, ointments, creams, suspensions, and the like as are well-known in the art. Systemic compositions include, but are not limited to, those designed for administration by injection, for example, subcutaneous, intravenous injection (IV), intramuscular injection (IM), intrathecal injection (IT), intraperitoneal injection (IP), as well as those designed for transdermal, subcutaneous, transmucosal oral, or pulmonary administration. For injection, the pharmaceutical compositions can be formulated in aqueous solutions, preferably in physiologically compatible buffers such as Hanks solution, Ringer's solution, or physiological saline buffer and/or in certain emulsion formulations. The solution can contain one or more formulatory agents such as suspending, stabilizing and/or dispersing agents. In certain examples the pharmaceutical compositions can be provided in powder form for constitution with a suitable vehicle, for example, sterile pyrogen-free water, before use. For transmucosal administration, one or more penetrants appropriate to the barrier to be permeated can be used in the formulation. Such penetrants are generally known in the art.

For oral administration, the pharmaceutical compositions can combine the beraprost with one or more pharmaceutically acceptable carriers well known in the art. Such carriers facilitate formulation as tablets, pills, dragees, capsules, liquids, gels, syrups, slurries, suspensions, and the like, for oral ingestion by a patient to be treated. For oral solid formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents may be added, such as the cross-linked polyvinylpyrrolidone, agar, or alginic acid or a salt thereof such as sodium alginate. If desired, solid dosage forms may be sugar-coated or enteric-coated using standard techniques.

For oral liquid preparations such as, for example, suspensions, elixirs and solutions, suitable carriers, excipients, or diluents include water, glycols, oils, alcohols, etc. Additionally, flavoring agents, preservatives, coloring agents and the like can be added. For buccal administration, the compositions may take the form of tablets, lozenges, etc. formulated in conventional manner.

For administration by inhalation, the pharmaceutical compositions can be delivered in the form of an aerosol spray from pressurized packs or a nebulizer, with the use of a suitable propellant, for example, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the compound and a suitable powder base such as lactose or starch.

In some examples, the pharmaceutical compositions are immediate release pharmaceutical compositions, modified release pharmaceutical compositions, or a combination thereof. In some examples, the immediate release pharmaceutical composition releases the beraprost within a short period of time after administration, typically less than about 4 hours, less than about 3.5 hours, less than about 3 hours, less than about 2.5 hours, less than about 2 hours, less than about 90 minutes, less than about 60 minutes, less than about 45 minutes, less than about 30 minutes, less than about 20 minutes, or less than about 10 minutes.

**In** some examples, the modified release composition may release the beraprost at a sustained or controlled rate over an extended period of time or may release it after a lag time after administration. For example, it may be released from the composition 4 hours after administration, 8 hours after administration, 12 hours after administration, 16 hours after administration, or 24 hours after administration. Modified release compositions include extended release, sustained release, and delayed release compositions. **In** some examples, the modified release compositions may release about 10% in about 2 hours, about 20% in 2 hours, about 40% in about 2 hours, about 50% in about 2 hours, about 10% in about 3 hours, about 20% in 3 hours, about 40% in about 3 hours, about 50% in about 3 hours, about 10% in about 4 hours, about 20% in 4 hours, about 40% in about 4 hours, about 50% in about 4 hours, about 10% in about 6 hours, about 20% in 6 hours, about 40% in about 6 hours, or about 50% in about 6 hours.

In some examples, modified release compositions may comprise a matrix selected from microcrystalline cellulose, sodium carboxymethylcellulose, hydroxyalkylcelluloses such as hydroxy propyl methylcellulose and hydroxypropylcellulose, polyethylene oxide, alkylcelluloses such as methylcellulose and ethylcellulose, polyethylene glycol, polyvinylpyrrolidone, cellulose acetate, cellulose acetate butyrate, cellulose acetate phthalate, cellulose acetate trimellitate, polyvinyl acetate phthalate, polyalkylmethacrylates, polyvinyl acetate and mixtures thereof.

The modified release compositions can also be formulated as a depot preparation. Such long acting formulations can be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

### Subjects to be treated

The subject can generally be any mammal. Examples of subjects include a non-human primate, a human, a dog, a cat, a mouse, a rat, a cow, a goat, a sheep, a rabbit, a horse, and a pig. In some examples, the subject is a human. The terms "subject," "individual" or "patient" are used interchangeably and as used herein are intended to include human and non-human animals. Non-human animals include all vertebrates, for example, mammals and non-mammals, such as non-human primates, sheep, dogs, rats, cats, cows, horses, chickens, amphibians, and reptiles. Examples of mammals include non-human primates, sheep, dogs, cats, cows, and horses. In some examples, the subject is a human or humans. The methods are suitable for treating humans having cancer. The subject may be symptomatic or asymptomatic.

### Kits

In additional examples, kits are provided for treating cancer in a subject. The kits can comprise a first container containing a pharmaceutical composition comprising at least an effective amount of prostacyclin/prostaglandin analog esuberaprost or pharmaceutically acceptable salt thereof; a second container containing CAR T-cells; and instructions for the administration of the pharmaceutical composition to the subject. Any of the above-described pharmaceutical compositions can be included in the kit. The kit can further comprise a third container, and so on containing additional pharmaceutical compositions or other active ingredients. In some examples, the first container can contain a pharmaceutical composition, a second container containing CAR T-cells, and a third container can contain at least one solvent or solvents to be mixed with the pharmaceutical composition before administering to the subject according to the instructions. In an example, the kit can comprise a first container containing beraprost or a pharmaceutically acceptable salt thereof, a second container containing CAR T-cells, and a third container containing an aqueous solvent.

### EXAMPLES

### Example 1: Evaluation of mouse model of CRS

This study will contain 3 groups - placebo control, positive control (tocilizumab or dexamethasone), and GP1681 treated. The GP1681 treated groups would have multiple arms for a dose-response determination in a murine model of CRS. These dosages will provide the information required for the secondary in vivo models of CAR-T therapy associated CRS treatment.

Humanized mice (expressing human PBMCs - HU-PBMC NSG^{™}; commercially available from The Jackson Laboratory; Bar Harbor, ME, USA) will be given either control treatments or 1 of 5 doses of GP1681 prior to CRS induction. To induce CRS, treated mice will be administered the antibody OKT3 intraperitoneally (IP), (anti-CD3 monoclonal antibody) to induce CRS. Mice will be sacrificed 24-48 hours post CRS-induction and cytokine production will be quantified (peripheral and in tissues).

Results will show that mice receiving GP1681 prior to CRS induction had lower undesired cytokine production than mice in the control group. Results will also show a reduction in the rapid, acute symptoms that occur in the mouse model within the first 48 hours. Longer models would show survival benefits, but this particular Example will not be conducted to that point.

### Example 2: Mouse model of CAR T-cell therapy induced CRS

This study will contain 3 groups -placebo control, dexamethasone control, and GP1681 dose determined from the primary CRS mouse studies of Example 1. SCID or humanized mice (expressing human PBMCs - HU-PBMC NSG^{™}) will be injected (IP) with Raji-luc tumor cells and observed for approximately three weeks for tumor growth. Tumor burden will be assessed via bioluminescence.

CAR-T cell treatment (IP) will be performed to solicit CRS (occurs approximately 2-3 days after CAR-T infusion). Controls and GP1681 treatments (IP, BID for 7 days) will start approximately 5 hours prior to CAR-T cell transfer. At the end of the 7-day treatment regimen tumor burdens will be assessed via bioluminescence and mice sacrificed for gross histopathology as well as peripheral and tissue cytokine concentrations determined.

Results will show that mice receiving either dexamethasone or GP1681 had lower undesired cytokine production than mice in the control group, and that GP1681 was superior to dexamethasone. While both the dexamethasone control and GP1681 will demonstrate a reduction in cytokine levels, it is expected that there will be greater survival benefits to GP1681 over dexamethasone.

### Example 3: Treatment with no delay period

A group of 50 human subjects having B-cell lymphoma will be divided into two groups - a control group and a GP1681 treatment group. Both groups will receive infusion of CAR T-cells. The GP1681 treatment group will receive GP1681 starting with co-administration with the CAR T-cells and continuing daily for seven days. Cytokines will be monitored daily for both groups. Results will show that subjects receiving GP1681 had reduced CRS symptoms and lower undesired cytokine production than subjects in the control group.

### Example 4: Treatment with predetermined delay period

A group of 80 human subjects having mantle cell lymphoma will be divided into two groups - a control group and a GP1681 treatment group. Both groups will receive infusion of CAR T-cells. The GP1681 treatment group will receive GP1681 starting three days after administration with the CAR T-cells and continuing daily for eleven days. Cytokines will be monitored daily for both groups. Results will show that subjects receiving GP1681 had reduced CRS symptoms and lower undesired cytokine production than subjects in the control group.

### Example 5: Treatment with monitored delay period

A group of 40 human subjects having acute myeloid leukemia will be divided into two groups - a control group and a GP1681 treatment group. Both groups will receive infusion of CAR T-cells. Cytokines will be monitored daily for both groups. The GP1681 treatment group will start to receive GP1681 upon detection of an increase in any one of cytokines IL6, IFN-y, and IL10. Treatment with GP1681 will continue daily for ten days. Results will show that subjects receiving GP1681 had reduced CRS symptoms and lower undesired cytokine production than subjects in the control group.

### Example 6: Kit for treatment of cancer

A box will be configured with a first container containing an effective amount of beraprost or a pharmaceutically acceptable salt thereof, and written instructions for the administration of the pharmaceutical composition and CAR T-cells to a subject. The instructions can be printed on paper and placed within the box or can be a hyperlinked website having the written instructions. The box is combined with a second container containing autologous or allogenic CAR T-cells. The box can optionally contain a third container containing water or an aqueous solution to dissolve the beraprost or a pharmaceutically acceptable salt thereof.

### Example 7: Assessment of cytokine release following GP1681 treatment

Cytokine release assays in normal human PBMC for analysis of GP1681 down regulation of cytokine production were developed and performed per the parameters evaluated in earlier pilot work. For the current assays PBMC from 5 healthy donors were procured.

The assay began with pretreatment of rested cells with either a positive control drug (dexamethasone) or GP1681. Following pre-treatment, cells were stimulated with either LPS or Poly (I:C). Twenty-four to forty-eight hours post-stimulation supernatants were collected and tested both for viability (48 hours) as well as concentrations of 29 different cytokines (24 hours.). Viability assessments confirmed the lack of GP1681-associated cell cytotoxicity. Statistical comparisons in individual donor results were conducted between all test groups within each donor group (between individual stimulant-treatment pairs) to determine efficacy of stimulation as well as any potential GP1681 effects on cytokine production. Additionally, statistical analysis was performed on pooled donor data for each cytokine stimulant-treatment pair to comprehensively determine cytokine suppression effects of GP1681 for each cytokine.

Both LPS and Poly (I:C) stimulated PBMC from all five donors but there were some noted variabilities. Both stimulants failed to induce significant stimulation (stimulated cells vs. unstimulated control cells) in various cytokines from various donors. Likewise, there were donor-specific variabilities in the level of stimulation notably more often in Donor 5 who had a lower level of stimulation across all cytokines.

While these noted variations in cytokine responses were an unpredictable, unintentional result, this phenomenon does adequately represent the naturally observed variation in human immune responses. Taken together both as individual donor and pooled responses, GP1681 treatment demonstrated significant reduction in 21 different cytokines detailed in the following Table, where the ** symbol indicates the 21 cytokines.

| | LPS | | Poly(I:C) | |
|---|---|---|---|---|
| Cytokine | DEX | GP1681 | DEX | GP1681 |
| ** IL-1α | YES | YES | YES | YES |
| ** IL-1β | YES | YES | YES | YES |
| IL-2 | YES | NO | YES | NO |
| ** IL-4 | YES | YES | NO | YES |
| ** IL-5 | YES | YES | YES | YES |
| IL-6 | YES | NO | YES | NO |
| IL-7 | N/A | N/A | N/A | N/A |
| IL-8 | NO | NO | NO | NO |
| ** IL-9 | YES | YES | YES | NO |
| IL-10 | YES | NO | YES | NO |
| ** IL-12 | YES | YES | YES | YES |
| ** IL-13 | YES | YES | YES | YES |
| ** IL-15 | YES | YES | YES | YES |
| ** IL-17 | YES | YES | YES | NO |
| ** IL-18 | YES | YES | YES | YES |
| ** TNFα | YES | YES | YES | YES |
| ** CCL2 (MCP-1) | YES | YES | NO | YES |
| ** CCL3 (MIP1α) | YES | YES | NO | NO |
| ** CCL5 (RANTES) | YES | YES | YES | YES |
| ** CXCL9 (MIG) | YES | YES | YES | YES |
| ** CXCL10 (IP-10) | YES | YES | YES | YES |
| ** IFN-γ | YES | YES | YES | YES |
| ** IFN-α2 | YES | YES | YES | YES |
| GCSF | NO | NO | NO | NO |
| ** GM-CSF | YES | YES | N/A | N/A |
| ** FGF | YES | YES | NO | NO |
| ** PDGF | YES | YES | YES | YES |
| VEGF | N/A | N/A | N/A | N/A |
| TGFB | NO | NO | NO | NO |

These 21 include 10 cytokines not previously identified by Gemmus Pharma, Inc. studies. Further, this work indicated that IL-2, IL-6, IL-8, and IL-10 signals were not significantly reduced in this ex vivo assay. It's important to note, that these four cytokines were identified as reduced by GP1681 treatment in Gemmus Pharma's in vivo influenza (H5N1) therapeutic studies. Moreover, IL-2, IL-6, and IL-10 were reduced by GP1681 treatment in current CytoAgents in vivo influenza (H1N1) studies. The differences in the observed reductions in in vivo work versus ex vivo work highlights an important aspect of the inherent differences in the assays with the caveat that a negative result in ex vivo does not always equate a negative result in vivo. These results can be surprising and unexpected at times.

The final cytokine measured in these assays not analyzed in previous Gemmus Pharma, Inc. work was IFNα. IFNα is a type I interferon that is tightly linked to the antiviral response of the immune system which is not generally associated with suppression through suppression of NFkB induction. GP1681 had a significant reduction of the very low levels of IFNα produced in this ex vivo system. This does not directly relate to suppression of IFNα antiviral activity (IFNα was not suppressed during earlier in vivo GP1681influeza treatment studies). Altogether, data from Gemmus Pharma, Inc studies and the present work here confirm that the production of approximately 25 different cytokines are down regulated through treatment with GP1681.

### Example 8: Cellular targets - normal human PBMC

Cytokine release assays were performed in normal human PBMC from five donors of mixed age, race, and gender. Normal human PBMC were obtained from Lonza's extensive catalog of cellular reagents. Cells were thawed according to manufacturer's instructions, washed in complete growth media, and assessed for viability using trypan blue staining. A stock cell solution of 2x10⁶ cells/mL was suspended in complete growth media for allocation into black-walled plate cell wells (2X10⁵/well) for the assay. Cells were rested at 37°C, 5% CO₂ for 1 hour.

### Example 9: Treatment, stimulation, cell harvest and cell viability measures

After resting the cells, 100 µl of GP1681 was added to appropriate wells at a final dosing concentration of 750µM. Vehicle and dexamethasone (final dosing concentration of 1µM) were also added at 100 µl respectively to their appropriate wells, and all were incubated at 37°C with 5% CO₂ for 15 minutes. For cell stimulation, 20 µL of the 1.0 ng/mL LPS solution or the 250µg/ml Poly (I:C) solution were added to appropriate wells according to the group designation. Twenty-four hours later 100 uL of supernatant were collected from all wells and stored for cytokine analysis. Approximately four hours before final harvest, AlamarBlue dye was added to all wells. At forty-eight hours post stimulation all samples were collected, and supernatants tested for cell viability. When added to cells, AlamarBlue is modified by the reducing environment of viable cells and becomes highly fluorescent. Hence, increased fluorescence after cell staining indicates increased viability. Alamar blue viability assessments demonstrated no cytotoxicity when cells were treated with GP1681 in Donors 1 - 5. Likewise, no signs of cytotoxicity were observed by the stimulation of cells with LPS or Poly (I:C).

### Example 10: Cytokine readouts, end results, and conclusions

Cytokines were assessed on 24-hour post-stimulation collected samples on a MAGPIX instrument using Luminex multiplex technology via multiplex kits from Millipore. Concentrations of each cytokine were assessed based on a standard curve. Sample values collected by the multiplex analysis below the limit of detection were not included in final determinations. Undiluted donor IL-6 concentrations were beyond the linearity of the assay and hence the valuations were too high for the typical standard curve. Therefore IL-6 samples were re-run at a 1:5 dilution. The determined cytokine concentrations (pg/ml) results of each cytokine were plotted as individual donor stimulant-treatment pairs. Statistical comparisons on all cytokines individual donor results were conducted between control groups via a one-way ANOVA with multiple comparisons. ANOVIA analyses were between the stimulated groups (LPS only, Poly(I:C) only) and the controls (media only, dex, and vehicle) to determine efficacy of stimulation and dexamethasone positive control, as well as any potential vehicle effect. An ordinary one-way t-test was then used to compare the vehicle group to the GP1681 stimulation to determine if there was an effect with the test item. The stimulant-treatment pairs of all five donors as pooled were plotted, single means to evaluate the overall efficacy of GP1681 cytokine suppression. Statistical analysis (Wilcoxon matched-pairs signed rank test) was performed on the pooled donor results for each cytokine plotted as well. Cytokine suppression end results were determined by evaluating both the pooled results as well as the individual donor results. GP1681 reduced 21 of the 29 cytokines analyzed in this work.

### Example 11: Observations from ex vivo treatment results

Effective stimulation of the PBMCs via LPS or Poly(I:C) was determined by comparison of the media control group to the stimulation group (non-treatment). Both mitogens successfully stimulated the majority of the cytokines, however there were variabilities both between donors and between cytokines. VEGF and IL-7 failed to stimulate consistently with either LPS or Poly(I:C) to a level above the lower limits of the standard curves and hence were not computed in the pooled analysis. The failure to stimulate is likely an artifact of the ex vivo assay which must be run with relatively short windows of observation as well as the lack of the complexities of a complete in vivo system. Additional cytokines while successfully stimulated, didn't achieve stimulation to a level of significant value as compared to unstimulated media wells. Statistically significant increases in either LPS or Poly(I:C) were detected in a minority of donors in the TGF-b, IL- 2 results. Statistical significance was not achieved in the Poly(I:C) groups for GM-CSF, IL-8, IL-17A, CXCL10/IP-10, or CCL5/RANTES. Variability between the donor's general levels of stimulation was also a notable observation. Overall Donor 5 had a much lower level of stimulation across all cytokines when treated with both Poly(I:C) and LPS. Due to this lack of stimulation, Donor 5 data was not included in pooled and final analysis for IL-2, IL-4, IL- 5, IL-9, IL-15, IL-12, IL-13, IL-17, IL-18, GM-CSF, and PDGF. Donor 5 stimulation for cytokines IL-10 and CXCL10/IP- 10 was above the lowest limit of detection but the values were magnitudes (1-3 logs) below the other 4 donors hence while Donor 5 data was not included in the pooled analysis for these two cytokines, individual results were still considered in final determinations for GP1681 efficacy. Similarly, Donor 1 TGF-b results were all below the lower limit threshold from the standard curve so data from this donor for this cytokine were not included in the overall pooled analysis of GP1681 efficacy. Donor 4 did have a notable lower level of overall stimulation in several cytokines (but not below the threshold for inclusion in calculations). Taken together these differences highlight the natural variation seen in generalized immune responses to mitogens within a population likely due to genetic differences between the subjects.

Much like the observations noted for the variabilities seen between groups and donors for successful stimulation, a similar unpredictability was observed with treatment group results within both stimulation methods and donors as well. GP1681 was capable of decreasing concentration in a few cytokines on which dexamethasone had no effect. Overall, GP1681 suppressed cytokine production in 21 of the 29 cytokines evaluated. Only three cytokines, GCSF, TGF-b and IL-8, experienced a lack of suppressed production with both GP1681 and dexamethasone treatments, despite successful stimulation in both LPS and Poly(I:C) induced cells. Likewise, in some instances dexamethasone failed to suppress cytokine production while GP1681 successfully reduced produced cytokine concentrations (Poly(I:C)-stimulated cells - IL-4, CCL2/MCP1), as well as the vice versa with GP1681 failing to suppress cytokine production while dexamethasone successfully reduced the cytokine concentrations (Poly(I:C)-stimulated cells - IL-17, IL-9). Furthermore, in some instances, both dexamethasone and GP1681 demonstrated significant reductions in cytokine production in one stimulation mitogen (LPS) but not the other (Poly(I:C)), as observed in FGF and CCL3/MIP1a. Undeniably, the complexities of assessing the study in its entirety, controls versus GP1681 in the two different stimulants, in five donors, and 29 different cytokines, are quite formidable. Ultimately, conclusions based on evaluations of the pooled and individual donor data indicate GP1681 suppression of cytokine production in both stimulation methods in the following cytokines - IL-1a, IL-1b, IL-4, IL-5, IL-12,p70, IL-13, IL-15, IL-18, TNFα, CCL2/MCP-1, CCL5/RANTES, CXCL9/MIG, CXCL10/IP-10, IFN-γ, IFNα,and PDGF; and observed suppression in cytokines from LPS stimulation alone - IL-9, IL-17, CCL3/MIP1a, GM-CSF, and FGF.

There are many operational factors to consider in the interpretation of the mechanisms and actions behind these tabulated results. Each stimulant triggers responses through different cellular receptors which in turn can then have different timing in the subsequent cellular cascade of events. Further, the window of analysis for this study - 24 hours, while necessary for the nature of this ex vivo study, likely does not provide enough time for all the evaluated cytokines' production to be initiated or reach a level of significant expression. Additionally, in lacking a complete biological system, recruitment of immunological factors that can play a role in the different cytokine responses are also not present in the entirety that exists in an intact physiological system. Hence negative results should be observed with this knowledge and require the awareness that in vivo results could be somewhat different. For instance, in this ex vivo study, IL-2, IL-6, IL-8 and IL-10 were all negative for GP1681 suppression. However, in concurrent data from in vivo lethal influenza H1N1 studies (and previous Gemmus Pharma Inc. H5N1 in vivo studies) GP1681 significantly suppressed the production of these cytokines.

GP1681 suppression of ex vivo IFNα activity was surprising and unexpected. IFNα is a type 1 interferon not typically associated with the same pathways or patterns of standard proinflammatory cytokines, or more pointedly, activity through NFkB induction. Type 1 interferons are an important part of the antiviral immune response. Further, type I interferons are not typically associated with NFkB production as IFNα is produced from different promoters and transcriptional elements than standard proinflammatory cytokines. The pathway for IFNα, from cellular receptors to feedback loops, is entirely different from proinflammatory cytokines. Inclusion of IFNα in this study was initially incorporated as a safety, not an efficacy measure. The results seen here are the reverse of the ex vivo/in vivo phenomenon mentioned above for IL-2, IL-6, et. al. While IFNα suppression by GP1681 is apparent in the data in these ex vivo studies, our in vivo data indicates the contrary, or no significant repression of IFNα concentrations by GP1681 treatment during lethal H1N1 influenza infections. Of importance when evaluating these two contrasting results is the recognition of the substantial difference in the amount of IFNα produced in ex vivo stimulated PBMC versus active viral replication induced IFNα levels in vivo. LPS stimulated IFNα production in PBMC was < 101 pg/ml, while Poly(I:C) stimulated levels were approximately 101pg/ml. H1N1 lethally infected mice had approximately 103 pg/ml IFNα detected in BALF fluids. The nominal reduction in the ex vivo production of IFNα (albeit statistically significant) was less than 2-fold. At the levels of IFNα produced during active viral replication, this small reduction would not be a significant change in BALF IFNα. IFNα production occurs in two phases, early and late, with the late production correlated with the high levels of IFNα associated with viral infections. One of the transcription factors responsible for a pronounced portion of IFNα production in the early phase is IRF7. IRF7 has an NFkB response element in its promoter. Mechanistically, this is the likely means that GP1681 indirectly influences IFNα production, having significant yet minimal in magnitude effects on the type 1 interferon produced as observed in this ex vivo assay.

In summary, this data and information demonstrate a broad efficacy of GP1681 cytokine suppression of 25 different cytokines identifying new cytokines (10 as compared to previous Gemmus Pharma, Inc. data) affected through ex vivo treatment of stimulated PBMC with the molecule.

In the above detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting.

## Claims

1. CAR-T cells and a pharmaceutical composition comprising at least an effective amount of BPS-314d (esuberaprost sodium salt) for use in a method of treating cancer in a subject, the method comprising administering CAR T-cells and the pharmaceutical composition to the subject.

2. CAR-T cells for use in a method of treating cancer in a subject, the method comprising administering CAR-T cells and a pharmaceutical composition comprising at least an effective amount of BPS-314d (esuberaprost sodium salt).

3. The CAR-T cells and pharmaceutical composition for use of claim 1 or the CAR-T cells for use of claim 2, wherein the subject:
a) experiences reduced cytokine release syndrome (CRS) relative to a similar subject receiving administered CAR T-cells but not receiving the administered pharmaceutical composition; or
b) does not experience CRS.

4. A pharmaceutical composition comprising at least an effective amount of BPS-314d (esuberaprost sodium salt) for use in a method of treating cytokine release syndrome (CRS) in a patient receiving CAR-T cell therapy.

5. The CAR-T cells and pharmaceutical composition for use of claim 1 or CAR-T cells for use of claim 2, wherein the cancer is B-cell lymphoma, aggressive, relapsed or refractory diffuse large B cell lymphoma, primary mediastinal B-cell lymphoma, high grade B-cell lymphoma, transformed follicular lymphoma, relapsed or refractory mantle cell lymphoma, acute lymphoblastic leukemia, Hodgkin's lymphoma, non-Hodgkin's lymphoma, acute myeloid leukemia, or multiple myeloma, brain cancer, breast cancer, glioblastoma, lung cancer, non-small-cell lung cancer, multiple myeloma, ovarian cancer, neuroblastoma, colorectal, biliary, pancreatic, mesothelioma, hepatoblastoma, embryonal sarcoma, prostate, sarcoma, or liver metastases.

6. The CAR-T cells and pharmaceutical composition, CAR-T cells or pharmaceutical composition for use of any of the preceding claims, wherein the subject is a mammal, non-human primate, cat, dog, pig, cow, goat, horse, sheep, or rabbit, optionally wherein the subject is a human.

7. The CAR-T cells and pharmaceutical composition, the CAR-T cells or the pharmaceutical composition for use of any of the preceding claims, wherein:
a) the CAR T-cells and the pharmaceutical composition are administered to the subject concurrently; or
b) the pharmaceutical composition is administered to the subject after the CAR T-cells are administered to the subject, optionally
i) wherein the pharmaceutical composition is administered to the subject starting about 1 day, about 2 days, about 3 days, about 4 days, about 5 days, about 6 days, or about 7 days after the CAR T-cells are administered to the subject; or
ii) wherein the pharmaceutical composition is administered to the subject starting about 3 days to about 7 days after the CAR T-cells are administered to the subject; or
c) wherein the pharmaceutical composition is administered to the subject before administration of the CAR T-cells.

8. The CAR-T cells and pharmaceutical composition, CAR-T cells or pharmaceutical composition for use of any of the preceding claims, wherein the pharmaceutical composition is administered to the subject:
a) after the CAR T-cells are administered to the subject; and
once onset of CRS is detected; or
b) after the CAR T-cells are administered to the subject; and
once onset of CRS is detected by an increased level of one or more of cytokine IL-1α, IL-β, IL-2, IL-4, IL-6, IL-7, IL-8, IL-10, IL-12, IFN-y, TNF-α, IP-10, MCP-1, MIP-1, RANTES, and GM-CSF; or
c) after the CAR T-cells are administered to the subject; and
once onset of CRS is detected by an increased level of one or more of cytokine IL-6, IL-10, IFN-y, and TNF-α.

9. The CAR-T cells and pharmaceutical composition, the CAR-T cells or the pharmaceutical composition for use of any of the preceding claims, wherein the pharmaceutical composition is administered for a period of about:
a) 1 day to about 30 days; or
b) 7 days to about 14 days.

10. The CAR-T cells and pharmaceutical composition, the CAR-T cells or the pharmaceutical composition for use of any of the preceding claims, wherein:
a) the CAR T-cells are autologous; or
b) the CAR T-cells are allogenic.

11. The CAR-T cells and pharmaceutical composition, CAR-T cells or pharmaceutical composition for use of any of the preceding claims, wherein the pharmaceutical composition:
a) further comprises at least one excipient, at least one filler, at least one disintegrant, at least one binder, at least one wetting agent, at least one lubricant, at least one glidant, at least one preservative agent, at least one flavoring agent, at least one antioxidant, or combinations thereof; or
b) is formulated as a tablet, capsule, granule, powder, liquid, suspension, gel, syrup, slurry, suppository, patch, nasal spray, aerosol, injectable, implantable sustained-release formulation, or mucoadherent film.

12. The CAR-T cells and pharmaceutical composition, CAR-T cells or pharmaceutical composition of any of the preceding claims, wherein the administering comprises topical delivery, subcutaneous delivery, intravenous injection (IV) delivery, intramuscular injection (IM) delivery, intrathecal injection (IT) delivery, intraperitoneal injection (IP) delivery, transdermal delivery, subcutaneous delivery, oral delivery, transmucosal oral delivery, pulmonary delivery, inhalation delivery, intranasal delivery, buccal delivery, rectal delivery, vaginal delivery, or combinations thereof.

13. The CAR-T cells and pharmaceutical composition, CAR-T cells or pharmaceutical composition of any of the preceding claims, wherein:
a) the BPS-314d (esuberaprost sodium salt) is present in a unit dose of the pharmaceutical composition in an amount of about 1 microgram to about 100 micrograms; or
b) the administering comprises delivering the pharmaceutical composition to the subject at an amount of BPS-314d (esuberaprost sodium salt):
i) at least about 0.1 microgram; or
ii) at about 0.1 microgram to about 5000 micrograms; or
iii) at about 15 micrograms to about 90 micrograms; or
iv) at about 60 micrograms to about 360 micrograms.

14. A kit comprising: a first container containing a pharmaceutical composition comprising at least an effective amount of BPS-314d (esuberaprost sodium salt); a second container containing CAR T-cells; and instructions for the administration of the pharmaceutical composition and CAR T-cells to a subject, optionally, further comprising a third container containing water or an aqueous solution.

## Patentansprüche

1. CAR-T-Zellen und pharmazeutische Zusammensetzung, umfassend mindestens eine wirksame Menge an BPS-314d (Esuberaprost-Natriumsalz), zur Verwendung in einem Verfahren zum Behandeln von Krebs bei einem Subjekt, wobei das Verfahren Verabreichen von CAR-T-Zellen und der pharmazeutischen Zusammensetzung an das Subjekt umfasst.

2. CAR-T-Zellen zur Verwendung in einem Verfahren zum Behandeln von Krebs bei einem Subjekt, wobei das Verfahren Verabreichen von CAR-T-Zellen und einer pharmazeutischen Zusammensetzung umfasst, die mindestens eine wirksame Menge an BPS-314d (Esuberaprost-Natriumsalz) umfasst.

3. CAR-T-Zellen und pharmazeutische Zusammensetzung zur Verwendung nach Anspruch 1 oder CAR-T-Zellen zur Verwendung nach Anspruch 2, wobei das Subjekt:
a) an einem reduzierten Zytokinfreisetzungssyndrom (CRS) im Vergleich zu einem ähnlichen Subjekt leidet, das verabreichte CAR-T-Zellen, aber nicht die verabreichte pharmazeutische Zusammensetzung erhält; oder
b) nicht an CRS leidet.

4. Pharmazeutische Zusammensetzung, umfassend mindestens eine wirksame Menge an BPS-314d (Esuberaprost-Natriumsalz) zur Verwendung in einem Verfahren zum Behandeln eines Zytokinfreisetzungssyndroms (CRS) bei einem Patienten, der eine CAR-T-Zell-Therapie erhält.

5. CAR-T-Zellen und pharmazeutische Zusammensetzung zur Verwendung nach Anspruch 1 oder CAR-T-Zellen zur Verwendung nach Anspruch 2, wobei der Krebs B-Zell-Lymphom, aggressives, rezidiviertes oder refraktäres diffuses großzelliges B-Zell-Lymphom, primäres mediastinales B-Zell-Lymphom, hochgradiges B-Zell-Lymphom, transformiertes follikuläres Lymphom, rezidiviertes oder refraktäres Mantelzell-Lymphom, akute lymphoblastische Leukämie, Hodgkin-Lymphom, Non-Hodgkin-Lymphom, akute myeloische Leukämie oder multiples Myelom, Hirnkrebs, Brustkrebs, Glioblastom, Lungenkrebs, nicht kleinzelliger Lungenkrebs, multiples Myelom, Eierstockkrebs, Neuroblastom, Kolorektal-, Gallen-, Bauchspeicheldrüsen-, Mesotheliom, Hepatoblastom, embryonales Sarkom, Prostata-, Sarkom oder Lebermetastasen ist.

6. CAR-T-Zellen und pharmazeutische Zusammensetzung, CAR-T-Zellen oder pharmazeutische Zusammensetzung zur Verwendung nach einem der vorhergehenden Ansprüche, wobei das Subjekt ein Säugetier, ein nichtmenschlicher Primat, Katze, Hund, Schwein, Kuh, Ziege, Pferd, Schaf oder Kaninchen ist, wobei das Subjekt optional ein Mensch ist.

7. CAR-T-Zellen und pharmazeutische Zusammensetzung, CAR-T-Zellen oder pharmazeutische Zusammensetzung zur Verwendung nach einem der vorhergehenden Ansprüche, wobei:
a) die CAR-T-Zellen und die pharmazeutische Zusammensetzung dem Subjekt gleichzeitig verabreicht werden; oder
b) die pharmazeutische Zusammensetzung dem Subjekt verabreicht wird, nachdem die CAR-T-Zellen dem Subjekt verabreicht wurden, optional
i) wobei die pharmazeutische Zusammensetzung dem Subjekt beginnend etwa 1 Tag, etwa 2 Tage, etwa 3 Tage, etwa 4 Tage, etwa 5 Tage, etwa 6 Tage oder etwa 7 Tage, nachdem die CAR-T-Zellen dem Subjekt verabreicht wurden, verabreicht wird; oder
ii) wobei die pharmazeutische Zusammensetzung dem Subjekt beginnend etwa 3 Tage bis etwa 7 Tage, nachdem die CAR-T-Zellen dem Subjekt verabreicht wurden, verabreicht wird; oder
c) wobei die pharmazeutische Zusammensetzung dem Subjekt vor der Verabreichung der CAR-T-Zellen verabreicht wird.

8. CAR-T-Zellen und pharmazeutische Zusammensetzung, CAR-T-Zellen oder pharmazeutische Zusammensetzung zur Verwendung nach einem der vorhergehenden Ansprüche, wobei die pharmazeutische Zusammensetzung dem Subjekt wie folgt verabreicht wird:
a) nachdem die CAR-T-Zellen dem Subjekt verabreicht wurden; und
sobald das Auftreten von CRS nachgewiesen wurde; oder
b) nachdem die CAR-T-Zellen dem Subjekt verabreicht wurden; und
sobald das Auftreten von CRS durch einen erhöhten Spiegel eines oder mehrerer von Zytokin IL-1α, IL-β, IL-2, IL-4, IL-6, IL-7, IL-8, IL-10, IL-12, IFN-γ, TNF-α, IP-10, MCP-1, MIP-1, RANTES und GM-CSF nachgewiesen wurde; oder
c) nachdem die CAR-T-Zellen dem Subjekt verabreicht wurden; und
sobald das Auftreten von CRS durch einen erhöhten Spiegel eines oder mehrerer von Zytokin IL-6, IL-10, IFN-γ und TNF-α nachgewiesen wurde.

9. CAR-T-Zellen und pharmazeutische Zusammensetzung, CAR-T-Zellen oder pharmazeutische Zusammensetzung zur Verwendung nach einem der vorhergehenden Ansprüche, wobei die pharmazeutische Zusammensetzung für etwa einen folgenden Zeitraum verabreicht wird:
a) 1 Tag bis etwa 30 Tage; oder
b) 7 Tage bis etwa 14 Tage.

10. CAR-T-Zellen und pharmazeutische Zusammensetzung, CAR-T-Zellen oder pharmazeutische Zusammensetzung zur Verwendung nach einem der vorhergehenden Ansprüche, wobei:
a) die CAR-T-Zellen autolog sind; oder
b) die CAR-T-Zellen allogen sind.

11. CAR-T-Zellen und pharmazeutische Zusammensetzung, CAR-T-Zellen oder pharmazeutische Zusammensetzung zur Verwendung nach einem der vorhergehenden Ansprüche, wobei die pharmazeutische Zusammensetzung:
a) ferner mindestens einen Hilfsstoff, mindestens einen Füllstoff, mindestens ein Sprengmittel, mindestens ein Bindemittel, mindestens ein Benetzungsmittel, mindestens ein Schmiermittel, mindestens ein Gleitmittel, mindestens ein Konservierungsmittel, mindestens einen Aromastoff, mindestens ein Antioxidans oder Kombinationen davon umfasst; oder
b) als Tablette, Kapsel, Granulat, Pulver, Flüssigkeit, Suspension, Gel, Sirup, Aufschlämmung, Zäpfchen, Pflaster, Nasenspray, Aerosol, injizierbare, implantierbare Formulierung mit verzögerter Freisetzung oder mucoadhäsiver Film formuliert ist.

12. CAR-T-Zellen und pharmazeutische Zusammensetzung, CAR-T-Zellen oder pharmazeutische Zusammensetzung nach einem der vorhergehenden Ansprüche, wobei das Verabreichen eine topische Abgabe, subkutane Abgabe, Abgabe mittels intravenöser Injektion (IV), Abgabe mittels intramuskulärer Injektion (IM), Abgabe mittels intrathekaler Injektion (IT), Abgabe mittels intraperitonealer Injektion (IP), transdermale Abgabe, subkutane Abgabe, orale Abgabe, transmukosale orale Abgabe, pulmonale Abgabe, Inhalationsabgabe, intranasale Abgabe, bukkale Abgabe, rektale Abgabe, vaginale Abgabe oder Kombinationen davon umfasst.

13. CAR-T-Zellen und pharmazeutische Zusammensetzung, CAR-T-Zellen oder pharmazeutische Zusammensetzung nach einem der vorhergehenden Ansprüche, wobei:
a) das BPS-314d (Esuberaprost-Natriumsalz) in einer Einheitsdosis der pharmazeutischen Zusammensetzung in einer Menge von etwa 1 Mikrogramm bis etwa 100 Mikrogramm vorliegt; oder
b) die Verabreichung Abgeben der pharmazeutischen Zusammensetzung an das Subjekt in einer folgenden Menge an BPS-314d (Esuberaprost-Natriumsalz) umfasst:
i) mindestens etwa 0,1 Mikrogramm; oder
ii) bei etwa 0,1 Mikrogramm bis etwa 5000 Mikrogramm; oder
iii) bei etwa 15 Mikrogramm bis etwa 90 Mikrogramm; oder
iv) bei etwa 60 Mikrogramm bis etwa 360 Mikrogramm.

14. Kit, umfassend: einen ersten Behälter, der eine pharmazeutische Zusammensetzung enthält, die mindestens eine wirksame Menge an BPS-314d (Esuberaprost-Natriumsalz) umfasst; einen zweiten Behälter, der CAR-T-Zellen enthält; und Anweisungen für die Verabreichung der pharmazeutischen Zusammensetzung und der CAR-T-Zellen an ein Subjekt, optional ferner umfassend einen dritten Behälter, der Wasser oder eine wässrige Lösung enthält.

## Revendications

1. Cellules CAR-T et composition pharmaceutique comprenant au moins une quantité efficace de BPS-314d (sel de sodium d'ésubéraprost) destinées à être utilisées dans une méthode de traitement du cancer chez un sujet, la méthode comprenant l'administration de cellules CAR-T et de la composition pharmaceutique au sujet.

2. Cellules CAR-T destinées à être utilisées dans une méthode de traitement du cancer chez un sujet, le procédé comprenant l'administration de cellules CAR-T et d'une composition pharmaceutique comprenant au moins une quantité efficace de BPS-314d (sel de sodium d'ésubéraprost).

3. Cellules CAR-T et composition pharmaceutique destinées à être utilisées selon la revendication 1 ou cellules CAR-T destinées à être utilisées selon la revendication 2, ledit sujet :
a) présentant un syndrome de libération de cytokines (CRS) réduit par rapport à un sujet similaire recevant des cellules CAR-T administrées mais ne recevant pas la composition pharmaceutique administrée ; ou
b) ne présentant pas de CRS.

4. Composition pharmaceutique comprenant au moins une quantité efficace de BPS-314d (sel de sodium d'ésubéraprost) destinée à être utilisée dans une méthode de traitement du syndrome de libération de cytokines (CRS) chez un patient recevant une thérapie par cellules CAR-T.

5. Cellules CAR-T et composition pharmaceutique destinées à être utilisées selon la revendication 1 ou cellules CAR-T destinées à être utilisées selon la revendication 2, ledit cancer étant un lymphome à lymphocytes B, un lymphome diffus à grands lymphocytes B agressif récidivant ou réfractaire, un lymphome à lymphocytes B médiastinal primaire, un lymphome à lymphocytes B de haut grade, un lymphome folliculaire transformé, un lymphome à cellules du manteau récidivant ou réfractaire, la leucémie lymphoblastique aiguë, un lymphome de Hodgkin, un lymphome non hodgkinien, la leucémie myéloïde aiguë ou un myélome multiple, le cancer du cerveau, le cancer du sein, un glioblastome, le cancer du poumon, le cancer du poumon non à petites cellules, un myélome multiple, le cancer de l'ovaire, un neuroblastome, colorectal, des voies biliaires, du pancréas, un mésothéliome, un hépatoblastome, un sarcome embryonnaire, de la prostate, un sarcome ou les métastases hépatiques.

6. Cellules CAR-T et composition pharmaceutique, cellules CAR-T ou composition pharmaceutique destinées à être utilisées selon l'une quelconque des revendications précédentes, ledit sujet étant un mammifère, un primate non humain, un chat, un chien, un porc, une vache, une chèvre, un cheval, un mouton ou un lapin, ledit sujet étant éventuellement un humain.

7. Cellules CAR-T et composition pharmaceutique, cellules CAR-T ou composition pharmaceutique destinées à être utilisées selon l'une quelconque des revendications précédentes :
a) lesdites cellules CAR-T et ladite composition pharmaceutique étant administrées au sujet simultanément ; ou
b) ladite composition pharmaceutique étant administrée au sujet après l'administration des cellule CAR-T au sujet, éventuellement
i) ladite composition pharmaceutique étant administrée au sujet environ 1 jour, environ 2 jours, environ 3 jours, environ 4 jours, environ 5 jours, environ 6 jours ou environ 7 jours après l'administration des cellules CAR-T au sujet ; ou
ii) ladite composition pharmaceutique étant administrée au sujet entre environ 3 jours et environ 7 jours après l'administration des cellules CAR-T au sujet ; ou
c) ladite composition pharmaceutique étant administrée au sujet avant l'administration des cellules CAR-T.

8. Cellules CAR-T et composition pharmaceutique, cellules CAR-T ou composition pharmaceutique destinées à être utilisées selon l'une quelconque des revendications précédentes, ladite composition pharmaceutique étant administrée au sujet :
a) après l'administration des cellules CAR-T au sujet ; et
une fois l'apparition du CRS détectée ; ou
b) après l'administration des cellule CAR-T au sujet ; et
une fois que l'apparition du CRS est détectée par un taux accru d'une ou de plusieurs des cytokines IL-1α, IL-β, IL-2, IL-4, IL-6, IL-7, IL-8, IL-10, IL-12, IFN-γ, TNF-α, IP-10, MCP-1, MIP-1, RANTES et GM-CSF ; ou
c) après l'administration des cellule CAR-T au sujet ; et
une fois l'apparition du CRS détectée par un taux accru d'une ou de plusieurs des cytokines IL-6, IL-10, IFN-γ et TNF-α.

9. Cellules CAR-T et composition pharmaceutique, cellules CAR-T ou composition pharmaceutique destinées à être utilisées selon l'une quelconque des revendications précédentes, ladite composition pharmaceutique étant administrée pendant une période d'environ :
a) 1 jour à environ 30 jours ; ou
b) 7 jours à environ 14 jours.

10. Cellules CAR-T et composition pharmaceutique, cellules CAR-T ou composition pharmaceutique destinées à être utilisées selon l'une quelconque des revendications précédentes :
a) lesdites cellules CAR-T étant autologues ; ou
b) lesdites cellules CAR-T étant allogènes.

11. Cellules CAR-T et composition pharmaceutique, cellules CAR-T ou composition pharmaceutique destinées à être utilisées selon l'une quelconque des revendications précédentes, ladite composition pharmaceutique :
a) comprenant en outre au moins un excipient, au moins une charge, au moins un délitant, au moins un liant, au moins un agent mouillant, au moins un lubrifiant, au moins un agent de glissement, au moins un agent conservateur, au moins un agent aromatisant, au moins un antioxydant, ou leurs combinaisons ; ou
b) étant formulée sous la forme d'un comprimé, d'une capsule, d'un granulé, d'une poudre, d'un liquide, d'une suspension, d'un gel, d'un sirop, d'une suspension, d'un suppositoire, d'un timbre, d'un spray nasal, d'un aérosol, d'une formulation injectable implantable à libération prolongée ou d'un film mucoadhésif.

12. Cellules CAR-T et composition pharmaceutique, cellules CAR-T ou composition pharmaceutique selon l'une quelconque des revendications précédentes, ladite administration comprenant une administration topique, une administration sous-cutanée, une administration par injection intraveineuse (IV), une administration par injection intramusculaire (IM), une administration par injection intrathécale (IT), une administration par injection intrapéritonéale (IP), une administration transdermique, une administration sous-cutanée, une administration orale, une administration orale transmuqueuse, une administration pulmonaire, une administration par inhalation, une administration intranasale, une administration buccale, une administration rectale, une administration vaginale ou des combinaisons de celles-ci.

13. Cellules CAR-T et composition pharmaceutique, cellules CAR-T ou composition pharmaceutique selon l'une quelconque des revendications précédentes :
a) ledit BPS-314d (sel de sodium d'ésubéraprost) étant présent dans une dose unitaire de la composition pharmaceutique en une quantité d'environ 1 microgramme à environ 100 microgrammes ; ou
b) ladite administration comprenant l'administration de la composition pharmaceutique au sujet à une quantité de BPS-314d (sel de sodium d'ésubéraprost) :
i) d'au moins environ 0,1 microgramme ; ou
ii) d'environ 0,1 microgramme à environ 5000 microgrammes ; ou
iii) d'environ 15 microgrammes à environ 90 microgrammes ; ou
iv) d'environ 60 microgrammes à environ 360 microgrammes.

14. Kit comprenant : un premier récipient contenant une composition pharmaceutique comprenant au moins une quantité efficace de BPS-314d (sel de sodium d'ésubéraprost) ; un deuxième récipient contenant des cellules CAR-T ; et des instructions pour l'administration de la composition pharmaceutique et des cellules CAR-T à un sujet, comprenant en outre éventuellement un troisième récipient contenant de l'eau ou une solution aqueuse.
